# EUROPEAN PATENT APPLICATION

(11) **EP 0 968 724 A1**
(43) Date of publication of application: **05.01.2000**
(21) Application number: 98202249.3
(22) Date of filing: 03.07.1998
(51) Int. Cl.: A61K 48/00, C12N 7/02

(54) **Use of viral vectors for treatment of the injured peripheral and central nervous system**

(71) Applicant: Koninklijke Nederlandse Akademie van Wetenschappen, 1011 JV Amsterdam (NL)
(72) Inventor: Anderson, Patrick Norval, Orpington, Kent BR5 1AA (GB); Zhang, Yi, Greenford, Middlesex UB6 0DX (GB); Dijkhuizen, Paul Alexander, 1054 BX Amsterdam (NL); Verhaagen, Joost, 1019 BX Amsterdam (NL); Hermens, Wilhelmus T. J. M. C., 1313 HE Almere (NL)
(74) Representative: Van Someren, Petronella F. H. M.

(57) **Abstract**

The present invention relates to the use of a viral vector other than wild type adenoviral vectors containing the gene for a nerve growth influencing molecule, for preparation of a therapeutical composition for the treatment of injuries in the peripheral or central nervous system, wherein the nerve growth influencing molecule is selected from the group consisting of neurotrophic factors, cell adhesion molecules, growth associated molecules and their receptors. The viral vector is selected from adeno-associated viral vectors (AAV) and adenoviral vectors, which adeno-associated viral vectors are preferably human adeno-associated vector-2 (AAV-2) and which adenoviral vectors are selected from the group consisting of first, second third or further generation adenoviral vectors.

The invention further relates to a method for concentrating adeno-associated viral vectors, comprising the step of banding the vectors in Iodixanol.

## Description

The present invention relates to the use of viral vectors for the treatment of injured nerves in the peripheral and central nervous system.

Both peripheral nerves and nerves in the spinal cord can be heavily damaged by for example motor cycle accidents. Spontaneous regeneration is either difficult or impossible. Therefore, the repair of nerve damage should be aided in some way.

Experimental and clinical approaches to promote regeneration of peripheral nerves have so far been based on neurosurgical and neuropharmacological treatment protocols. Current neurosurgical procedures pertain to reimplantation of avulsed ventral nerve roots, direct suture of the proximal and distal nerve stump of the interrupted nerve, the use of peripheral nerve autografts to bridge the gap between two nerve stumps, and the application of artificial, synthetic or biodegradable nerve guide tubes to restore the original nerve tract.

These neurosurgical techniques have considerably improved recovery following peripheral nerve trauma in humans, but less than 10 to 25% of the patients undergoing surgical nerve repair exhibit complete recovery of function. Local or systemic administration of several compounds (e.g. gangliosides, neuropeptides, vitamin B, and nimodipine) have been shown to accelerate peripheral nerve regeneration in animal models but the outcome of clinical studies have been disappointing and so far none of these molecules is currently used as a pharmacotherapy for human peripheral nerve damage.

Injury to dorsal roots in the spinal cord, particularly those connected to the brachial plexus, is another serious clinical problem which is also difficult to treat. In adult mammals following dorsal root injury the central processes of primary sensory (DRG) neurons regenerate successfully in the peripheral nervous tissue of the dorsal roots but stop abruptly at the dorsal root entry zone (DREZ) in which CNS glial cells are present. The DREZ represents a highly non-permissive environment for axonal regeneration, so that the regenerating axons either turn back into the root or form swollen endbulbs amongst the DREZ astrocytes. The regeneration of the central processes of DRG neurons can be enhanced by a conditioning injury to their peripheral processes, following which small numbers of axons succeed in growing back into the superficial dorsal horn but most are still unable to reenter the spinal cord.

In view of the above described problems it is the object of the present invention to provide a new means to treat injured nerves, both at the periphery and in the spinal cord.

According to the invention there is thus provided the new use of viral vectors containing the gene for a nerve growth influencing molecule for the preparation of a therapeutical composition for the treatment of injured nerves.

In the research that led to the present invention it has been demonstrated that an adenoviral vector can be used to efficiently direct the expression of a foreign gene in Schwann cells of intact or injured rat peripheral nerves. Initially adenoviral vectors harboring LacZ (Ad-LacZ) were used as a model system. Following infusion of 8x10⁷ pfu Ad-LacZ, a large number of both myelinating and ensheathing Schwann cells along a stretch of 9 to 12 mm of the sciatic nerve displayed β-gal expression. β-gal expression persisted in many Schwann cells for at least 12 days post-infusion.

It has furthermore been demonstrated according to the invention that regeneration of the dorsal roots of the spinal cord can be achieved by means of gene therapy with a viral vector coding for neurotrophine-3. The dorsal roots of the 4th, 5th and 6th lumbar spinal nerves were severed and reanastomosed in adult rats and, 14 to 19 days later, adenoviral vectors containing either the lacZ or NT-3 genes were injected into the lumbar spinal cord. The vectors produced strong expression of the transgenes in glial cells and motor neurons around the injection tract from 4 to 40 days after injection. When the central processes of primary sensory neurons were transganglionically labelled with HRP conjugated to cholera toxin subunit B, 16 to 37 days after dorsal root injury, large numbers of sensory axons could be seen to have regenerated back into the cord, but only in those animals injected with vector carrying the NT-3 gene. The regenerated axons were found at the injection site, mainly in the grey matter, and penetrated as deep as lamina V.

The invention thus for the first time demonstrates that it is possible to achieve regeneration of injured nerves into the spinal cord by means of gene therapy.

According to the invention the nerve growth influencing molecule that is encoded in the viral vector is a nerve growth stimulating molecule selected from neurotrophic factors, cell adhesion molecules, growth associated proteins and their receptors.

Examples of neurotrophic factor are brain/derived neurotrophic factor (BDNF), neurotrophin-3 (NT-3), neurotrophic growth factor (NGF), glia-derived neurotrophic factor (GDNF), acidic and basic fibroblast growth factor (FGF).

Cell adhesion molecules can be selected from Li, neural/cell adhesion molecule (N-CAM), Axonin-1/TAG-1 and cadherins, whereas the growth associated proteins can be the intrinsic neuron growth cone proteins B-50/GAP-43 (growth/associated protein with an apparent molecular weight of 43 kD).

As an alternative to the nerve growth influencing molecule, receptors for these molecules can be introduced in the injured cells by gene therapy. That way, the treatment is not effected through the nerve growth influencing molecule directly, but indirectly. By overexpressing the receptors for these types of molecules there are provided more sites for binding the influencing molecule, which can indirectly lead to a higher growth influencing activity in the cells. Those receptors can be selected from members of the tyrosine-kinase receptor familie, trkA, trkB and trkC.

The injury to the nerves that can be treated by the use of viral vectors according to the invention can be the result of a variety of events, such as dorsal or ventral root avulsions or contusion of the spinal cord. Furthermore, the injury can lead to atrophy of nerve cells in brain nuclei giving rise to large descending spinal nerve tracts. Atrophy of nerve cells can also be treated by gene delivery with adenoviral vectors encoding neurotrophic factors.

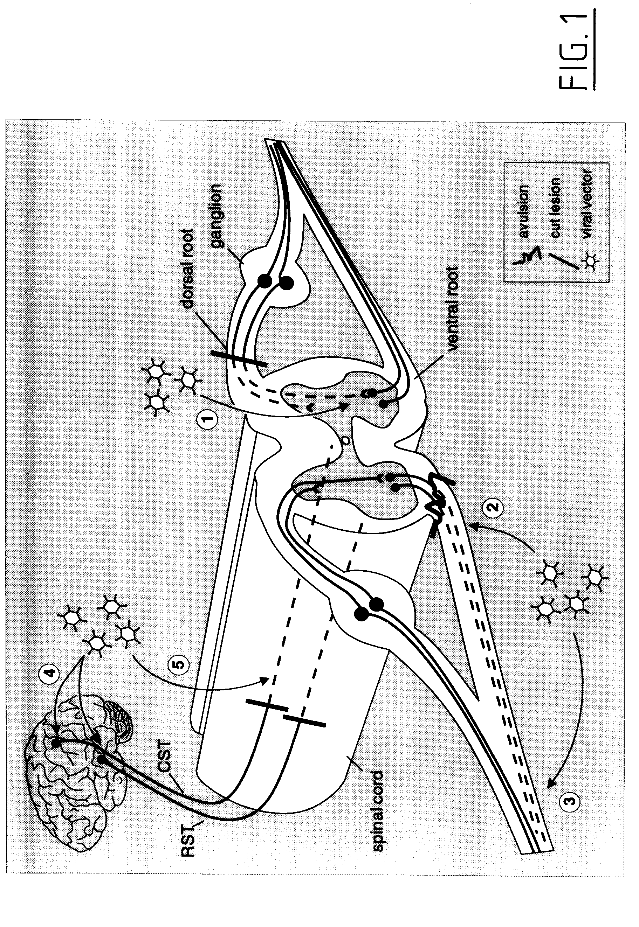

Figure 1 shows schematically to what locations the viral vectors of the invention can be applied to lead to repair of nerve damage. In Figure 1 the reference numeral 1 refers to the treatment of dorsal root avulsions. At 2 ventral root avulsions are shown. The numeral 3 refers to peripheral nerve injuries. 4 indicates atrophied brain nuclei that are involved in ascending and descending nerve paths of the spinal cord. 5 refers to injuries inside the spinal cord.

One embodiment of the invention is concerned with the treatment of peripheral nerve injury. Most peripheral nerve injuries are associated with changes in Schwann cell phenotype. For transduction of Schwann cells located in the distal part of a crushed nerve using a viral vector three approaches of vector delivery to a crushed sciatic nerve can be distinguished. In two approaches involving the infusion of the vector simultaneously with or immediately after the nerve crush, a relatively small number of transduced Schwann cells is found. In a third preferred approach, the vector is administered one day after the nerve crush. This results in a large number of transduced Schwann cells equivalent to the transduction observed in uncrushed nerves injected with the same dosage of vector.

In nerves injected the day after the crush, regenerating nerve fibers grow in close association with transduced Schwann cells following vector infusion, showing that regeneration proceeds along the transduced Schwann cells according to a normal time course of peripheral nerve regeneration. Thus, Schwann cells transduced with a viral vector can support the extension of newly formed regenerating peripheral nerve sprouts.

The present invention thus shows that it is feasible to use Schwann cells of an injured peripheral nerve to overexpress or ectopically express molecules, such as neurotrophic factors, that could enhance peripheral nerve regeneration. The main hurdle for application of neurotrophic factors in neuroregeneration is the difficulty of delivering these proteins to the site where they should exert their action. Systemic or intrathecal administration is associated with unwanted side-effects, due to interactions with peripheral receptor sites. The delivery of neurotrophic factors through viral vector-mediated gene transfer according to the invention offers a novel approach to ensure sustained delivery of these factors that is restricted to the immediate environment of the regenerating nerve fibers. One preferred neurotrophic factors for use to enhance peripheral neuroregeneration is NT-3.

In another embodiment cell-adhesion molecules are locally expressed to guide and stimulate the initial stages of peripheral nerve regeneration. These molecules mediate the attachment of axons to Schwann cells and present a promoting cue for growth cone advancement. Cell adhesion molecules such as L1, N-CAM and N-Cadherin can stimulate neurite outgrowth in vitro. Overexpression of these molecules on the surface of Schwann cells in vivo can be used to enhance the supporting properties of Schwann cells on neurite extension.

Moreover, the technology of the invention could be applied to chronically injured nerves where it is critical to find ways to induce the sprouting response at the lesion site. The current approach could also prove useful in regeneration paradigms involving the repair of peripheral nerve roots or the use of peripheral nerve tissue bridges. First, overexpression of a neurotrophic factor within a peripheral nerve graft used to bridge a lesion gap, can result in attraction of more regrowing fibers. Second, following ventral root avulsion, the severed rootlets can be injected with a viral vector according to the invention prior to reimplantation into the spinal cord.

In a second embodiment the invention is involved with the treatment of dorsal and ventral root avulsions by gene therapy with viral vectors carrying a gene encoding a nerve growth influencing molecule. In particular it has been shown that gene therapy with vectors carrying neurotrophin genes offers an approach to otherwise intractable spinal root injuries. The dorsal roots of the 4th, 5th and 6th lumbar spinal nerves were severed and reanastomosed in adult rats and, 14 to 19 days later, adenoviral vectors containing the NT-3 gene were injected into the lumbar spinal cord. The vectors produced strong expression of the transgene in glial cells and motor neurons around the injection tract from 4 to 40 days after injection. When the central processes of primary sensory neurons were transganglionically labelled with HRP conjugated to cholera toxin subunit B, 16 to 37 days after dorsal root injury, large numbers of sensory axons could be seen to have regenerated back into the cord in animals injected with vector carrying the NT-3 gene. The regenerated axons were found at the injection site, mainly in the grey matter, and penetrated as deep as lamina V. Gene therapy with adenoviral vectors encoding a neurotrophin has therefore been shown to be capable of enhancing the regeneration of a subpopulation of dorsal root axons, (probably myelinated A fibres) back into the spinal cord.

In order to use gene therapy technology to restore the function of injured dorsal roots several further steps can be required. First, vectors carrying NGF and possibly BDNF can be used in addition to the NT-3 vector, in order to promote the regeneration of other classes of primary afferent fibre into the cord. The utility of the invention has been shown herein by reference to adenoviral vectors. However, according to a preferred embodiment of the invention a vector with less cytotoxity than the adenovirus is used since motor neurons might be lost when using adenoviral vectors as a result of the immune response elicited by the vectors. Adeno-associated virus vectors and lentiviral vectors are particularly preferred.

The viral vector is thus preferably selected from adeno-associated viral (AAV) vectors and adenoviral vectors, preferably modified adenoviral vectors. Adeno-associated viral vectors are preferably human AAV2 vectors.

When adenoviral vectors are used they may be first generation adenoviral vectors. Especially preferred however are modified adenoviral vectors selected from second, third en further generation adenoviral vectors. First generation adenoviral vectors were replication defective through deletions in the E1-region. However, it was found that after transduction there could still arise an accumulation of viral gene products that led to cytopathic effects on the transduced cells and activation of the immune system, possibly caused by the presence of cellular trans-activators with E1-like activity. The advantage of further generation vectors is thus that they are safer for use in humans and animals.

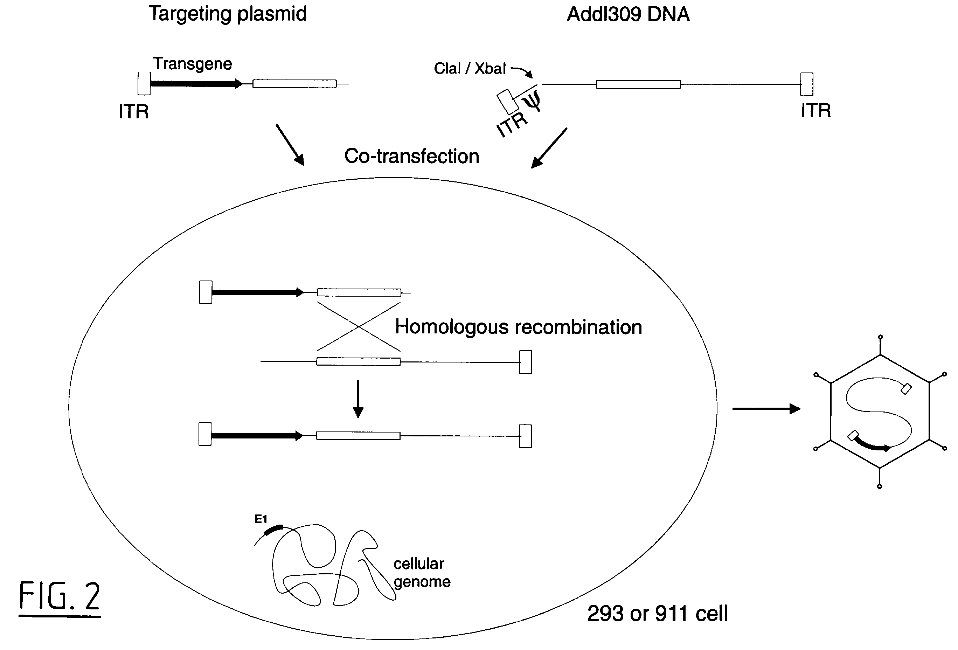

The present invention relates in particular to adeno-associated vectors harboring either one of the genes encoding NGF, NT-3, BDNF and GAP-43. Fig 2 shows the generation of a rAd vector. The targeting plasmid contains two cis-acting sequences of Ad type 5 (Ad5) essential for replication and packaging, the Ad5 replication origin located in the ITR and the packaging signal (the "Ψ" sequence), a transgene expression cassette, and a fragment of the Ad5 genome (map units 9.2-15.5) required for homologous recombination. The 5'ITR and Ψ sequence of Addl309 genomic DNA are removed by restriction with Cla I and Xba I to render the Ad genome replication and packaging defective. The genomic fragment is co-transfected with the targeting plasmid in producer cells in culture (293 or 911 cells). Homologous recombination between the targeting plasmid and the genomic Ad DNA results in a rAd vector. This genome can be replicated and packaged into infectious Ad vector particles since the producer cells constitutively express the E1 gene that is deleted in the rAd vector, but which function is essential for Ad vector replication. rAd vector particles can be harvested from the cells when a cytopathic effect is observed. rAd vectors can be purified and concentrated after cell lyses by CsCl density centrifugation.

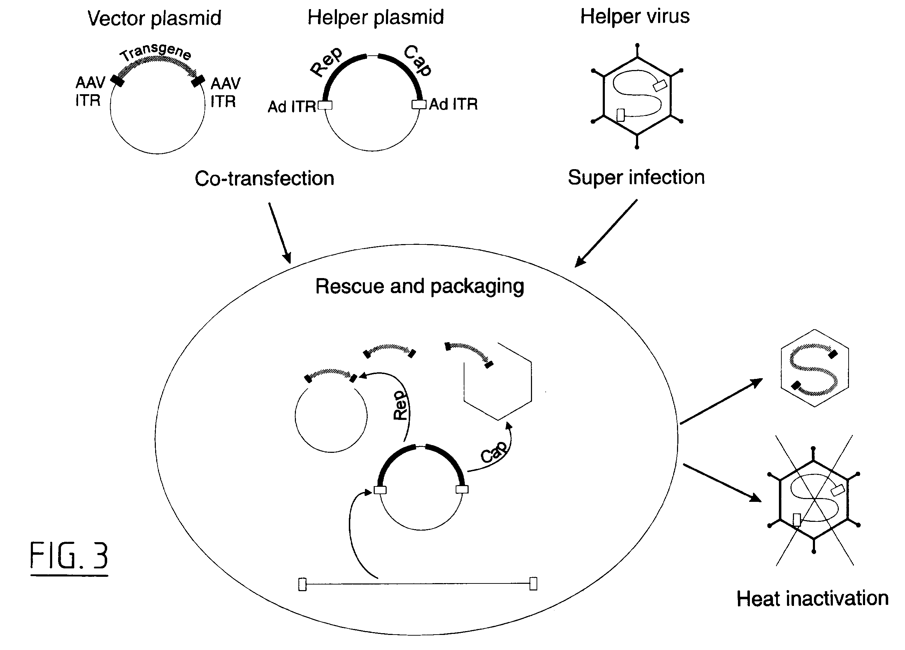

Fig. 3 shows generation of defective AAV vector stocks. A plasmid vector is constructed containing the cis-acting sequences of AAV type 2 (AAV2) essential for rescue, replication, and packaging, the AAV ITR's (AAV ITR), and flanking a transgene expression cassette. This plasmid vector and a helper plasmid containing the AAV Rep and Cap genes flanked by Ad ITR's (Ad-ITR) are co-tranfected into 293 cells. After the transfection the cells are superinfected with helper virus (Addl309). The Ad infects the cell and viral gene products trans-activate the Ad ITR's on the helper plasmid enhancing the production of Rep and Cap genes. The Rep genes products recognize the AAV ITR's on the plasmid vector, rescue the vector DNA from the plasmid and starts to replicate it. The produced Cap proteins form the AAV capsid and package the newly generated defective AAV genomes. After two days, when a cytopathic effect is observed, a mixed population of helper virus and defective AAV-vector particles can be obtained from the producer cells. The contaminating Ad can be inactivated by heating the stock at 56°C for 30 min. Defective AAV stocks can be concentrated and purified from cel debris and adenovirus by CsCl density centrifugation.

According to a further aspect thereof the invention relates to a method for concentrating adeno-associated viral vectors, comprising the step of banding the vectors in Iodixanol. The chemical name of Iodixanol is 5,5'-[(2-hydroxy-1,3-propanediyl)-bis(acetylimino)]bis-[N,N'-bis(2,3-dihydroxypropyl)-2,4,6-triiodo-1,3-benzenedicarboxamide]. Iodixanol is known as an iso-osmotical nonionic dimeric hydrophilic contrast agent and according to the invention was found to be very suitable for the highly efficient concentration of adeno-associated vectors. Iodixanol was found to concentrate adeno-associated viruses more efficiently than the commonly used cesium chloride. Another advantage of Iodixanol is that it is directly suitable for use in the human and animal body. Thus, an additional dialysis can be avoided before administering the concentrated viral vectors. During dialysis vectors could be lost.

In the examples that follow the adenoviral vector-mediated gene delivery to injured rat peripheral nerve and the delivery of NT-3 by an adenoviral vector, which induced injured dorsal root axons to regenerate back into the spinal cord are demonstrated. In addition protocols are given for testing the efficacy of various viral vector in inducing regeneration of injuries in various regions of the nervous system.

In the Example reference is made to the following figures:
Figure 1 shows schematically the locations for gene therapy in the peripheral and central nervous system.
Figure 2 is a schematic representation of the generation of an adenoviral vector harboring a transgene of interest.
Figure 3 is a schematic representation of the generation of an adeno-associated vector harboring a transgene of interest.

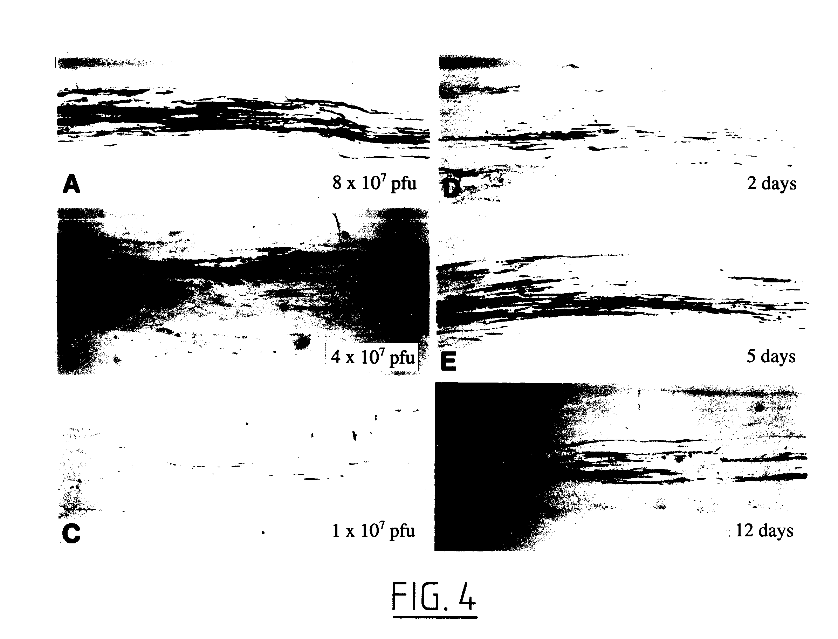

Figure 4 shows that Schwann cells of a peripheral nerve can be infected efficiently with an adenoviral vector but is dependent on the infused dosage of the viral vector. When 8x10⁷ pfu Ad-LacZ was infused into the sciatic nerve, a large number of β-gal positive Schwann cells were observed along a stretch of 9 to 12 mm five days after Ad-LacZ administration (a). The infusion of 4x10⁷ resulted in the transduction of fewer Schwann cells (b), while infusing 1x10⁷ led to the transduction of only a few Schwann cells (c). Analyzing the time course of β-gal expression in nerves infused with 8x10⁷ pfu Ad-LacZ revealed that many Schwann cells expressed the transgene 2 days after adenoviral vector administration (d), although most Schwann cells were not completely filled with the β-gal protein at this timepoint. Five days after Ad-LacZ infusion, many β-gal positive were observed (e). Twelve days after Ad-LacZ administration, many Schwann cells were still β-gal positive (f), although the number has decreased somewhat compared with five days after administration (e). Scale bar = 500 µm (a-f).

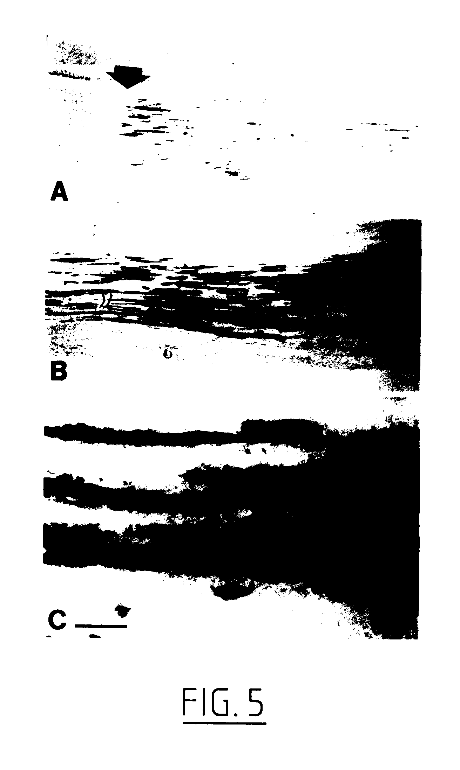

Figure 5 shows that when the sciatic nerve was crushed and 8x10⁷ pfu Ad-LacZ infused simultaneously, only a few transduced Schwann cells were observed (a). The distal border of the crush was clearly demarcated by a few β-gal positive Schwann cells (arrow). However, when 8x 10⁷ pfu Ad-LacZ was infused the day after the nerve crush, many transduced Schwann cells were present distal to the crush site (b). Typical morphological changes of Schwann cells following a peripheral nerve crush were revealed by β-gal histochemistry (c). Scale bar = 500 µm (a, b); 50 µm (c).

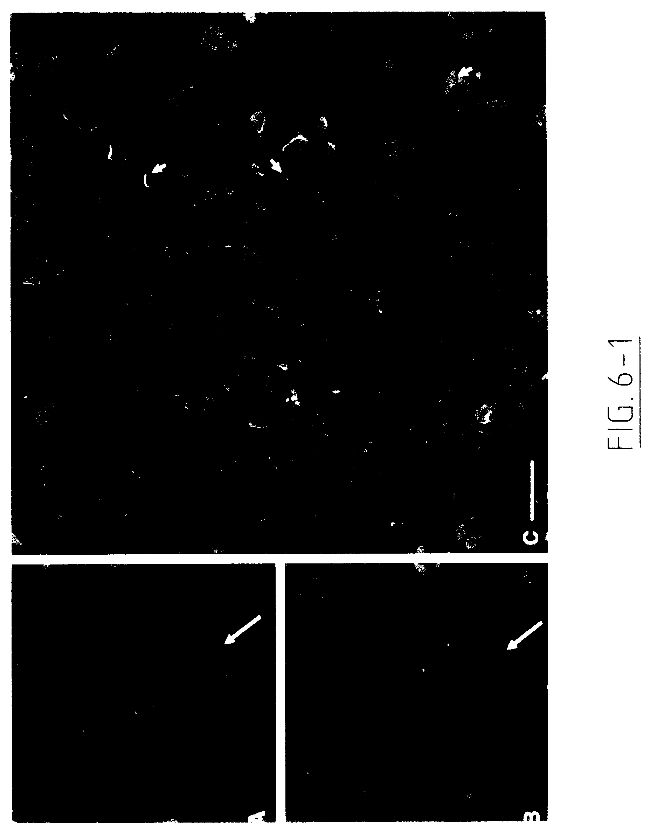

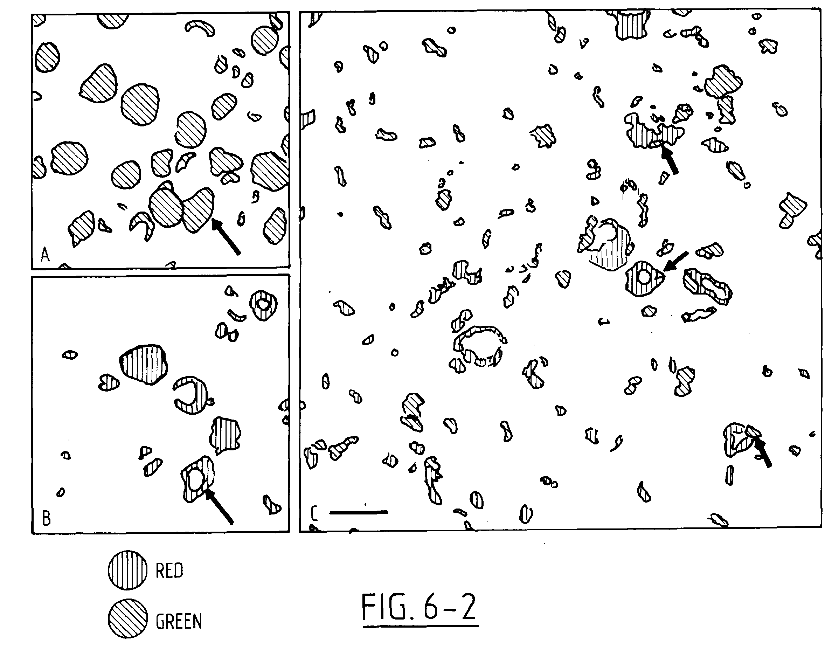

Figure 6 shows confocal images of double immunostaining for the Schwann cell marker S-100 (Fig. 6a; FITC optics) and β-gal (Fig. 6b; Cy3 optics) on transverse sections of a nerve injected with Ad-LacZ the day after a crush. There is a marked colocalization of β-gal and S-100 protein, indicating that β-gal positive cells are Schwann cells. Arrows in Fig. 6a and Fig. 6b point to an example of a myelinating Schwann cell that is outlined both on the basis of β-gal and S-100 immunoreactivity. Fig. 6c shows a double immunostaining for neurofilament 150 kD (FITC optics) and β-gal (Cy3 optics). Clearly many newly formed neurites were closely associated with β-gal positive Schwann cells (short arrows). This indicates that following transduction, Schwann cells retained their capacity to guide regenerating fibers. Scale bar for Fig. 6a and Fig. 6b is 15µm, for Fig. 6c 10 µm.

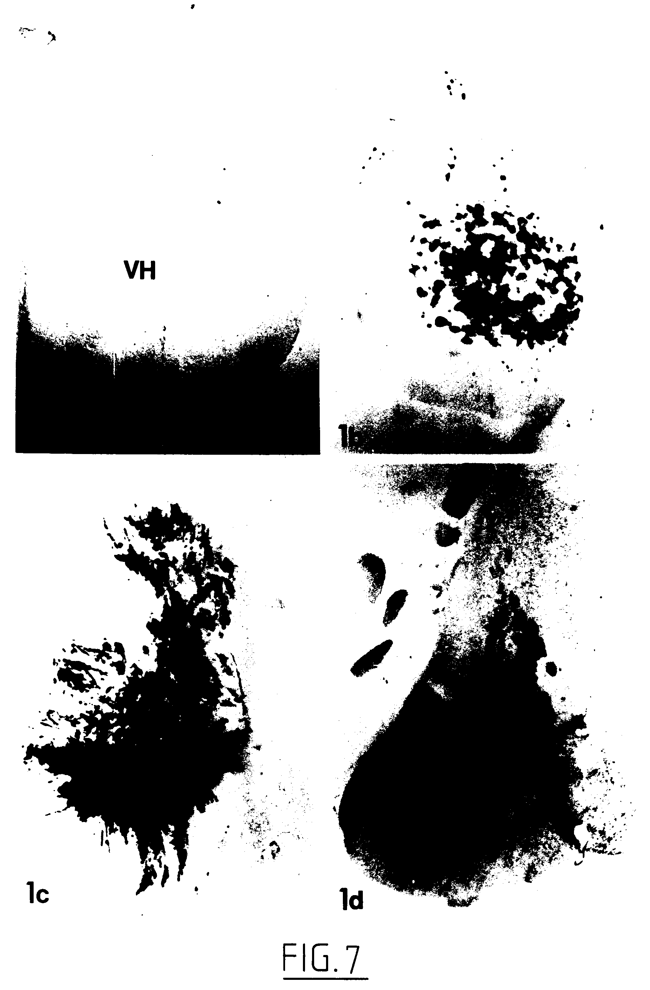

Figure 7 shows transverse sections of rat spinal cords processed for the visualization of NT-3 mRNA by in situ hybridization (Figures 7a and 7b) X-gal histochemistry (Figures 7c and 7d). The spinal cord illustrated are from animals 4 days (Figures 7b and 7c) and 12 days (Figure 7d) following Ad-NT3 (Figure 7b) injection or AdLacZ injection (Figures 7c, 7d). Figure 7a shows absence of NT-3 message on the uninjected side of the spinal cord. Figure 7b shows that many cells, including both large putative motor neurons and smaller glial cells, were infected by Ad-NT3 and expressed high levels of NT-3 mRNA. In Figure 7c and Figure 7d both motor neurons and glial cells express high levels of Lac-Z transgene at 4 days (Figure 7c) and 12 days (Figure 7d) following AdLacZ injection. VH = ventral horn. Scale bar = 500 µm (applies to Figures 7b-7d).

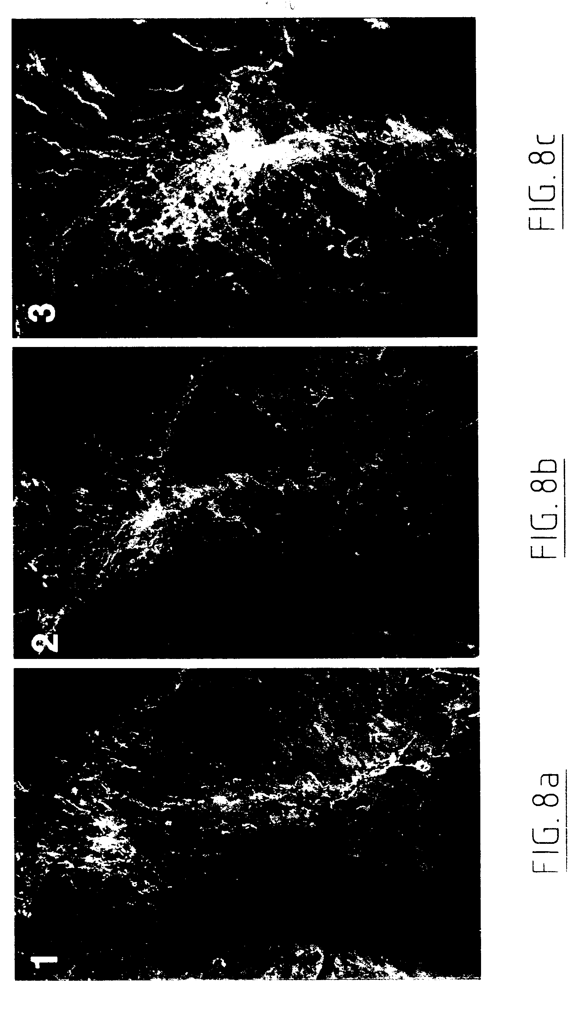

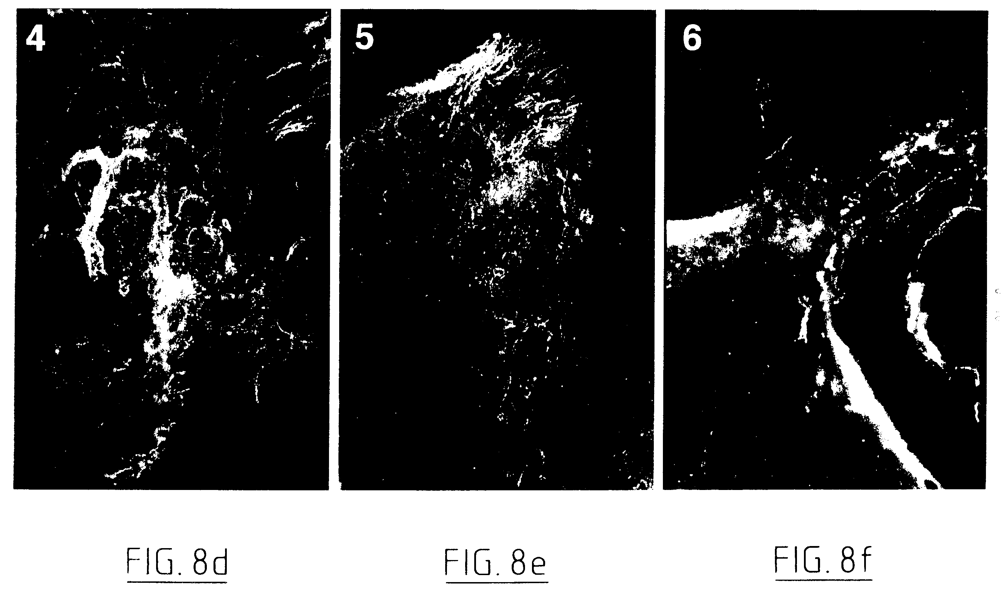

Figure 8 shows transverse sections of the dorsolateral part of the spinal cord showing regenerating axons transganglionically labelled with HRP in the dorsal root and/or dorsal horn 19 days (Figures 8b-8d) or 27 days (Figures 8f) following injections of Ad-NT3 (Figures 8b-8d) or AdLacZ (Figure 8f) into the left ventral horn of the segments of L4,5 spinal cord. The left L4,5,6 dorsal roots were sectioned and reanastomosed 17 days before injection with Ad-NT3 (Figures 8b-8f) and 19 days before injection with AdLacZ (Figure 8f). The diagram in Figure 8a shows the locational orientation of the photomicrographs. In Figure 8b numerous transganglionically labelled axons are present in the dorsal root and some of them have grown into the spinal cord. Note that almost all the labelled axons in the dorsal root appear to be arrested at the DREZ. Figure 8c shows HRP labelled axons growing directly into the cord. Figure 8d shows labelled axons growing into the cord apparently around blood vessels. In Figure 8e HRP labelled axons are seen extending into the deep parts of the dorsal horn (lamina V). In Figure 8f labelled axons are present in the dorsal root, but all appear to be arrested at the DREZ, no labelled axons can be seen growing into the cord. DR = dorsal root; DH = dorsal horn; DH = dorsal column; Scale bars = 100 µm.

### EXAMPLES

### EXAMPLE 1

### Introduction

Disturbances in the continuity of a peripheral nerve by a nerve crush or nerve cut results in the denervation of muscles and skin distal to the lesion. The first signs of degeneration in the distal nerve segment are observed as early as a few hours post-lesion, and proceed during the following post-lesion days. Features of the degeneration process include disintegration of interrupted axons, breakdown of myelin and the dedifferentiation and proliferation of Schwann cells within the distal nerve stump. After an initial delay, the formation and outgrowth of regenerating neurites starts. This regenerative sprouting response is essential for the eventual recovery of function of the damaged nerve.

The purpose of the present example is to explore the ability of adenoviral vectors to direct the expression of a foreign gene to crushed rat sciatic nerves. It is demonstrated that infusion of a recombinant adenoviral vector encoding the Escherichia coli β-galactosidase (LacZ) gene directs efficient beta-galactosidase (β-gal) expression to Schwann cells and ensheathing cells of intact and crushed rat peripheral nerves. A single injection of adenoviral vector resulted in transduced Schwann cells along a stretch of nerve approximately 1 cm long and robust transgene expression was observed up to 12 days after adenoviral vector administration. Regenerating nerve fibers grew unimpeded into the distal nerve stump, along transduced Schwann cells. The present example suggests that direct gene transfer may provide a powerful molecular genetic method to express nerve outgrowth promoting proteins within injured peripheral nerves in order to promote their capacity to regenerate.

### 2. Materials and methods

### 2.1 Adenoviral vector

The construction of a recombinant adenoviral vector carrying a LacZ expression cassette has been described previously (Hermens et al., J. Neurosci. Meth. 71: 85-98 (1997); Hum.Gene Ther. 8: 1049-1063 (1997)). In short, a plasmid was constructed that contains a CMV-LacZ expression unit, flanked upstream by the adenovirus type 5 (Ad5) inverted terminal repeat (ITR, map units 0-1.25) and downstream by map units 9.2-15.5 of the adenovirus genome.

This plasmid was linearized with SalI and co-transfected with ClaI and XbaI restriction digested Ad5d1309 DNA (Hermens et al., J. Neurosci. Meth. 71: 85-98 (1997); Hum.Gene Ther. 8: 1049-1063 (1997)). into 911 producer cells (Fallaux et al., Hum. Gene Ther. 7: 215-222 (1996)). Recombinant plaques were isolated, plaque purified, expanded on 911 cells and purified by two rounds of CSCI banding (Graham and Prevec, in: Methods in Molecular Biology, vol 7, Gene Transfer and Expression Protocols (E.J Murray, Ed.), pp 109-129 (1991); Hermens et al., J. Neurosci. Meth. 71: 85-98 (1997); Hum.Gene Ther. 8: 1049-1063 (1997)).

The recombinant adenovirus was dialyzed against TS (25 mM Tris, 137 mM NaCl, 6 mM KCl, 0.7 mM Na₂HPO₄, 1.1 mM MgCl₂, 0.9 mM CaCl₂; pH 7.4) and stored in TS with 10% glycerol at -80°C. The titer was determined by a plaque assay on 911 cells, and is expressed as plaque forming units (pfu) per ml.

### 2.2 Animal treatment

In all experiments, male Wistar rats (200-250 g) were used. Animals were anaesthetized with a combination of Hypnorm™ (0.03 ml/100g) and Dormicum™ (0.06 ml/100g). At the level of the hip, the right sciatic nerve was exposed, and the tibial branch was gently separated from the peroneal branch. A 30 gauge needle, fitted to a Hamilton syringe was inserted approximately 1 mm longitudinally into the tibial nerve and 1 gl Ad-LacZ solution containing 1x10⁷, 4x10⁷ or 8x10⁷ pfu was gently infused. The sciatic nerve was crushed according to a procedure that has been described previously (De Koning et al., J. Neurol. Sci. 74: 237-256 (1986)).

For the administration of Ad-LacZ to crushed sciatic nerves, three approaches were used. In the first approach, the sciatic nerve was crushed for 30 seconds using hemostatic forceps and simultaneously, 1 µl containing 8x10⁷ pfu Ad-LacZ was infused in the direction of the crush, 1 cm distal from the crush-site. In the second approach, the sciatic nerve was crushed and immediately following the crush, 1 µl 8x10⁷ pfu Ad-LacZ was infused. In the third approach, the right sciatic nerve was crushed and the following day, 1 µl containing 8x10⁷ pfu Ad-LacZ was infused 1 cm distally from the crush site.

### 2.3 Histology

At 2, 5, or 12 days following Ad-LacZ administration, animals were decapitated. The sciatic nerves were dissected and processed as described previously (Verhaagen et al., Brain Res. 404: 142-150 (1987)). Briefly, sciatic nerves were fixed for 2 to 3 h in either periodate-lysine-paraformaldehyde in 50 mM phosphate buffer, pH 7.5 (McLean and Nakane, J. Histochem. Cytochem. 22: 1077-1083 (1977)), for immunohistochemistry, or 4% paraformaldehyde in 50 mM phosphate buffer for X-Gal histochemistry. Sciatic nerves were cryoprotected by immersion in 15% and 30% sucrose in 50 mM phosphate buffer, frozen in dry ice cooled isopentane and cut transversally (10 pm thick sections) or longitudinally (25 gm thick sections) by a cryostat.

To detect β-galactosidase expression, sections were stained with X-Gal staining solution (5 mM K₄Fe(CN)₆, 5 mM K₃Fe(CN)₆, 2 mM MgCl₂ with 1 mg/ml 5-bromo-4-chloro-3-indolyl-D-galactoside) for 3 hr at 37°C, dehydrated in a graded ethanol series and mounted in Entallan (Merck).

For double immunohistochemistry the following primary antibodies were used: a monoclonal anti-β-gal antibody (gal-13, 1:2000; Sigma), an antiserum that specifically recognizes the neurofilament M subunit (4394, 1:100) (Verhaagen et al.J. Neurosci. Res. 16: 589-600 (1986); Verhaagen et al., Brain Res. 404: 142-150 (1987); Van Leeuwen, in Molecular Neuranatomy (Van Leeuwen, Buijs, Pool and Pach, Eds.), Elsevier Science Publishers (1988)) or a polyclonal anti S-100 antibody (Sigma; 1:200).

Sections were blocked in TBS containing 2% horse serum and 0.3% Triton X-100, followed by an incubation with the primary antibodies diluted in THZT (50 mM Tris-HCl, pH 7.5, 0.5 M NaCl, 0.5% Triton X-100) for 24 hr at room temperature. After 3 washes in TBS, sections were incubated with FITC-coupled swine-anti-rabbit (1:50; Dako) and Cy3-coupled Donkey-anti-mouse (1: 100; Jackson Immuno Research Laboratories) in THZT for 1 hr, washed 3 times with TBS and mounted in Vectashield mounting medium (Vector Laboratories). Double stained sections were analyzed using a confocal scanning laser microscope (Zeiss). Micrographs of longitudinal sections are composed of multiple scans spanning the depth of the section. Micrographs of transverse sections represent a scan in a single plane.

### 3. Results

### 3.1 Administration of Ad-LacZ to control sciatic nerves

To determine whether Schwann cells could be transduced efficiently with a recombinant adenoviral vector, intact sciatic nerves of adult Wistar rats were infused with three different dosages of Ad-LacZ (8x10⁷, 4x10⁷ or 1x10⁷ pfu, all in 1 µl). Five days later, the expression of β-gal in these nerves was detected by X-Gal histochemistry. The largest proportion of β-gal-positive Schwann cells (approximately 15%) was observed in nerves infused with a dose of 8x10⁷ pfu Ad-LacZ (n=5).

Expression was seen along a stretch of 0.9 to 1.2 cm (Figs. 4a and 4e). When nerves were infused with a dose of 4x10⁷ pfu Ad-LacZ (n=4), fewer Schwann cells were β-gal positive and these cells were present along a shorter stretch of the nerve (Fig. 4b). Infusion of 1x10⁷ pfu Ad-LacZ (n=4) resulted in the transduction of only a few Schwann cells (Fig. 4c) and the staining intensity was also much lower as compared to the higher dosages used. Because of these results, a dose of 8x10⁷ was used in all further experiments.

Next the time-course of β-gal expression in the intact sciatic nerve was determined. Many transduced Schwann cells were already apparent 2 days after infusion of Ad-LacZ. Similar numbers of Schwann cells appeared to be transduced as compared to 5 days post-infusion, but the intensity of staining of individual cells was much less and was not present throughout the cell. This suggests that it takes several days for the transgene product to accumulate throughout the cytoplasm of the Schwann cell. Twelve days after adenoviral vector administration, many Schwann cells still expressed the transgene. However, about 1.5-fold fewer positive cells were present than at the day 5 time point (Fig. 4f).

At all time points examined, both myelinating and ensheathing Schwann cells in the nerve expressed β-gal. The β-gal protein, localized in the cytoplasm, revealed the characteristic features of myelinating Schwann cells, in which the cytoplasm that extends in longitudinal bands and in transverse trabeculae forming bridges between the longitudinal bands. Ensheathing Schwann cells have a slender appearance and do not contain myelin sheaths.

### 3.2 Transduction of Schwann cells with Ad-LacZ in crushed sciatic nerves

Three different approaches were examined to determine the effectiveness of an adenoviral vector to transduce Schwann cells of a sciatic nerve following a crush injury. In all cases, 8x10⁷ pfu Ad-LacZ was injected 1 cm distal from the crush site.

In the first experimental setup, the sciatic nerve was crushed for 30 sec with hemostatic forceps, followed immediately by the infusion of Ad-LacZ solution (n=5).

In the second experimental setup, the sciatic nerve was crushed for 30 see with hemostatic forceps and simultaneously, the Ad-LacZ solution was infused (n=8).

In a third approach, the sciatic nerve was crushed and Ad-LacZ was infused the following day (n=7). In all cases, animals were analyzed 5 days following Ad-LacZ administration.

Infusion of Ad-LacZ immediately following the crush, resulted in virtually no transduction of Schwann cells 5 days after viral vector administration (not shown). However, when Ad-LacZ was infused simultaneously with the crush, a few β-gal positive Schwann cells were observed. Transduced Schwann cells were located between the crush site and the site of injection (Fig. 5a). The crush site was clearly identified, as the distal border of the crush was demarcated by β-gal positive Schwann cells. Nevertheless, crushed nerves that received a simultaneous viral vector injection, displayed significantly less transduced cells than the intact nerve injected with the same dosage of Ad-LacZ.

Infusion of 1 µl Ad-LacZ (containing 8x10⁷ pfu) 1 day following nerve crush resulted in many β-gal positive Schwann cells 5 days after Ad-LacZ administration (Fig. 5b). Compared with the infusion of 8x10⁷ pfu Ad-LacZ in the intact nerve, there was no obvious difference between the number of β-gal positive Schwann cells. Transgene expressing Schwann cells were present along a stretch of nerve similar in length as observed in adenoviral vector injected control nerves. Figures 6a and 6b confirm that β-gal positive cells are indeed Schwann cells. Double staining of transverse sections with an anti-β-gal antibody and an anti-S-100 antibody revealed a colocalization for β-gal and S-100 immunoreactivity.

Transduced Schwann cells retained the capacity to guide regenerating nerve fibers, as evidenced by a double immunostaining using a β-gal antibody and an antibody reacting with the 150 kDa neurofilament protein (antibody 4394). This antibody specifically recognizes a well defined N-terminal epitope of neurofilament M subunit (NF-M) (Verhaagen et al., J. Neurosci. Res. 16: 589-600 (1986)) and stains newly formed neurites, but does not react to neurofilament debris present in degenerating axon profiles (Verhaagen et al., Brain Res 404: 142-150 (1987)). As shown in confocal scanning micrographs, regenerating neurites were observed in close association with β-gal positive Schwann cells (Fig. 6c). Thus, in crushed nerves injected with an adenoviral vector, the newly formed neurites grew along the transduced, demyelinating Schwann cells, through the so-called Bands of Buengner, as can be observed in a non-injected nerve.

### EXAMPLE 2

### NT-3 delivered by an adenoviral vector induces injured dorsal root axons to regenerate back into the spinal cord

### 1. Introduction

The sensory axons in the dorsal roots of adult mammals are normally unable to regenerate into the spinal cord after injury. This example demonstrates that gene therapy with vectors carrying neurotrophin genes offers an approach to otherwise intractable spinal root injuries. Gene therapy with adenoviral vectors encoding a neurotrophin is shown to be capable of enhancing the regeneration of a subpopulation of dorsal root axons, (probably myelinated A fibres) back into the spinal cord.

Neurotrophin-3 (NT-3) is a trophic factor for large diameter 1a primary afferent neurons whose central processes normally end deep in the grey matter on motor neurons. Even in experiments with both a dorsal root injury and a conditioning peripheral nerve lesion, 1a afferents do not regenerate beyond laminae I and II of the spinal grey. In adult mammals, motor neurons express very low levels of NT-3 and can therefore offer little trophic or tropic stimulation to regenerating dorsal root axons. A source of NT-3 in the deep grey matter of the spinal cord is therefore expected to enhance the regeneration of 1a afferents, but is difficult to achieve by direct application of the neurotrophin. In this example an adenoviral vector is used to deliver NT-3 into glial cells and motor neurons of the spinal cord to promote the regeneration of NT-3 sensitive primary sensory axons into the spinal cord.

### 2. Materials and methods

Adenoviral vectors were constructed containing the rat NT-3 cDNA (Ad-NT3) or LacZ cDNA (Ad-LacZ) under control of the CMV promoter (P.A. Dijkhuizen et al., J. Neurobiol. 33, 172 (1997)). The bioactivity of Ad-NT3 was tested on embryonic DRG explants in vitro (Dijkhuizen et al., supra). The stability of infection and transgene expression in vivo was investigated by injecting the vectors into the spinal cords of adult female Sprague-Dawley rats (200-300 g).

The animals were deeply anaesthetized with halothane and the L4,5 segments of spinal cord were exposed by laminectomy. The dura was cut using fine microsurgical scissors. The adenoviral vector injections were performed with a fine glass needle 70 µm in diameter, fixed in a stereotaxic frame and attached via a polyethylene tube to a 10 µl Hamilton syringe. Each animal was given two injections of adenovirus encoding NT-3 or lacZ (control). The two injection sites were immediately to the left of the central vein, to a depth of 1.5 mm into the cord and separated rostral-caudally by about 5mm within the L4,5 segments of the spinal cord.

The adenoviral vectors were slowly injected into the spinal cord and the needle was left in place for a further minute before withdrawal from the cord. Dexamethasone was injected intramuscularly shortly before the virus injection and daily for three days after virus injection in each group. This treatment enhances the stability of transgene expression by suppressing the immune response.

Transgene expression was assessed between 4 and 40 days following injection. Sections of the spinal cord were used for 5-bromo-4-chloro-3-indolyl-D-galacto-pyranoside (X-Gal) staining and/or in situ hybridization for NT-3 mRNA. Both X-Gal staining and NT-3 in situ hybridization showed that many glial cells and some motor neurons within 2.5 mm of the injection sites expressed very high levels of the transgenes (Figs. 7a, 7b). Transgene expression was maximal between 4 and 12 days after injection (Figs. 7b), but had declined at 40 days (not shown).

The efficacy of Ad-NT3 in promoting NT-3 sensitive primary axonal outgrowth was assessed in adult female Sprague-Dawley rats (200-300 gm) in which the left L4, 5, 6 dorsal roots, which contribute to the sciatic nerve, were injured. Animals were anaesthetized with halothane and oxygen. The left lumbar dorsal roots and ventral roots were exposed by a hemilaminectomy. The L4, 5, and 6 dorsal roots were individually transected about 15 mm from their entrance into the cord and reanastomosed under an operating microscope by using 10/0 microsutures.

Bovine fibrinogen was used to ensure additional stability of the anastomosis. In order to prevent the retrograde labelling of motor neurons in the final phase of the experiments, the L4, 5, 6 ventral roots were also sectioned in some experiments. In order to enhance the regeneration of the central processes of primary sensory neurons within the spinal cord the left sciatic nerves were crushed several times at mid-thigh level with a pair of watchmakers' forceps.

Fourteen to nineteen days after dorsal root injury the animals were deeply re-anaesthetized with halothane, the L4,5 segments of spinal cord were exposed by laminectomy and the adenoviral vector injections were performed as above. The left sciatic nerve was again crushed several times with a pair of watchmakers' forceps, at the same level as the original crush.

Three groups of animals were examined. In group one (n=8), animals were injected with 0.5 µl of vehicle containing 1x10⁷ pfu Ad-LacZ. In group two (n=4) animals were injected with 0.5 µl of vehicle containing 1x10⁷ pfu Ad-NT3. In group three (n=3) animals were injected with 0.5 µl of a mixture containing 7.5x10⁶ pfu Ad-NT3 and 2.5x10⁷ pfu Ad-LacZ. In all 3 groups dexamethasone (5mg/kg) was injected intramuscularly before the virus injection and daily for three days thereafter.

Sixteen to thirty seven days after virus injection the animals were re-anaesthetized and 0.5 µl of 1.4% cholera toxin subunit-B conjugated HRP (CT-HRP) was injected into the left sciatic nerve using a 5 µl Hamilton syringe. Two to three days after CT-HRP injection the animals were perfused transcardially with 100 ml of 0.1 M phosphate buffer followed by 500 ml of 1% paraformaldehyde and 1.25% glutaraldehyde in 0.1 M phosphate buffer. The L4,5,6 segments of spinal cord were identified by tracing the sciatic nerve into the dorsal roots and into the dorsal horn, and removed. Transverse section were cut at 40-50 µm on a freezing microtome and collected into 0.1 M phosphate buffer. The free-floating sections were processed for the visualization of HRP using the tetramethyl benzidine method (M. M. Mesulam, Tracing neural connections with horseradish peroxidase (IBRO Handbook Series: Methods in the Neurosciences; Wiley-Interscience (1982)).

Nineteen (n=2), 26 (n=1), 33 (n=2) and 39 days (n=2) after Ad-NT3 or a mixture of Ad-NT3 and Ad-LacZ was injected into the spinal cord, numerous transganglionically labelled axons were found in the injured dorsal roots (Fig. 8b). Large numbers of such labelled axons were identified in the dorsal horn of the spinal cord at the Ad-NT3 injection sites (Fig. 8c).

Many of the labelled axons which could be traced into the spinal cord from the injured dorsal roots avoided passing through the DREZ (Fig. 8b). Commonly, the labelled axons could be seen growing into the spinal cord from its dorsal or dorsolateral surfaces along blood vessels (Fig. 8d). These axons were densely concentrated in and around the injection tract (Fig. 8e) but some extended up to 800pm into the grey matter and the lateral white column, although they did not extend significantly into the ventral horn (Fig. 8e), even though motor neurons and glia in that region were transfected. In the dorsal horn labelled axons formed plexuses in lamina II to IV, and some were found in lamina V. There was no evidence for the rostral growth of axons in the dorsal columns, an observation which confirms that the myelinated tracts in the CNS are usually inhibitory to regenerating axons.

In contrast, very few central axons of primary sensory neurons were able to grow into the spinal cord in the animals injected with Ad-LacZ (Fig. 8f, group one) at 16 (n=1), 19-20 (n=2), 27 (n=1), 33-34 (n=2), 40 (n=1), 47 (n=1) days after virus injection, and those that did penetrated no deeper than lamina I and II of the dorsal horn. Clearly, the slight damage caused by injection of the control vector was not sufficient to allow dorsal root fibres to regenerate into the cord in large numbers.

These results demonstrate for the first time that an increased level of NT-3 expression, resulting from an Ad vector injected into the ventral horn, promotes the regeneration of the central axons of primary sensory axons deep into the grey matter of the spinal cord. The observation that regenerating axons were concentrated within 0.8 mm of the area found to show NT-3 transgene expression indicates that over-expression of NT-3 not only acts as a trophic stimulus promoting regeneration into the cord but also acts as a tropic stimulus, attracting NT-3-sensitive axons.

### EXAMPLE 3

### Injection of viral vectors in various regions of the nervous system

Adenoviral vectors encoding a reporter gene (lacZ) or a neurotrophic factor or growth-associated protein (B50/GAP43) were tested in the following manner. Any other vector of the invention can be tested in the same way.

### 1. Peripheral nerve injections

At the level of the hip, the right sciatic nerve is surgically exposed, and the tibial branch is gently separated from the peroneal branch. A 30 gauge needle, fitted to a Hamilton syringe is inserted approximately 1 mm longitudinally into the tibial nerve and 1 µl of the viral vector solution containing 8x10⁷ pfu is gently infused.

### 2. Spinal cord injections

The spinal cord is exposed and a hemilaminectomy is performed on the T13 and L1 vertebra. The dura is cut with microsurgical scissors. Viral vector infusions are performed with a fine glass needle 70 µm in diameter, fixed in a stereotactic frame and attached via a polyethylene tube to a 10 µl Hamilton syringe. The needle is inserted 1 mm deep into the spinal cord and 0.5 µl buffer containing 1x10⁷ pfu is slowly infused and the needle is left in place for a further minute. The muscles and the skin are sutured.

### 3. Ventral root injections

The spinal cord is exposed and a hemilaminectomy is performed on the L1 and L2 vertebra. The dura is cut, and the L4, 5 and 6 ventral roots are exposed. A small glass needle is inserted longitudinally into the ventral root and 1 µl viral vector solution containing 8x10⁷ pfu is gently infused.

### 4. Motor cortex and Red Nucleus injections

Direct stereotactic injections (coordinates according to Paxinos and Watson (in: The rat brain in stereotaxic coordinates, Academic press, London (1982)) were performed under deep anesthesia with Hypnorm™ (0.1 ml/100g body weight, i.m.; Janssen Pharmaceutical, Ltd.) in the motor cortex (coordinates: AP-1.5, L+1.2, DV-2.5), and Red Nucleus (coordinates: AP-6.0, L+0.7, DV-6.5). The injections were performed with a stainless 30 G needle in a stereotactic apparatus attached via polyethylene tubing to a 10 µl Hamilton syringe in a Harvard 22 microinjection pump. Slow infusion of 0.2 µl (0.02 µl/mm) to 1.0 µl (0.1 µl/min) of Ad-LacZ (1x10¹¹pfu/ml) was performed.

### 5. Dissection and transduction of intercostal nerves

Adult rats were anaesthetized with isofluorane (1.6 %), and an incisure was made from axilla to the xiphoid process to expose the lateral and anterior cutaneous branches of the intercostal nerves. Eight nerves of approximately 1 cm were dissected and placed in Leibowitz (L-15) medium until further processing. The skin was closed using silk sutures.

Immediately after dissection of the intercostal nerves, the nerves were cleaned from fat tissue and washed in L15 medium, using a Zeiss dissecting microscope. The nerves were then fixated with insect pins onto sterile dental wax (Aurion) to stretch the nerves and make injection possible. Ex vivo micro-injection of the intercostal nerves was performed using glass capillaries, fitted to a Hamilton syringe with a plastic tube. Approximately 1 µl of recombinant virus (containing 10⁸ plaque forming units (pfu)) was injected in the dissected branches of the intercostal nerves. To visualize the flow of virus into the dissected nerves, 1 % fast green (Sigma) was added to the virus solution. The nerves were washed in L-15 medium, transferred to Dulbecco's Modified Eagle Medium and placed in a humidified chamber at 37°C until further processing.

### 6. Implantation of transduced peripheral nerve in the spinal cord

Rats were anaesthetized with isofluorane and the spinal cord was exposed at the low-thoracic level (T10) by a dorsal laminectomy. The midline was identified under a Zeiss operating microscope and the dura opened. With microscissors, an incision was made until the grey matter of the spinal cord appeared as a distinctive color. From this point, the lesion was expanded to the sides, thereby affecting corticospinal and rubrospinal. tracts. With this method, a three mm long dorsal hemisection of the spinal cord was performed, using a pasteur's pipet for gentle aspiration and micro scissors. Bleeding was stopped using thrombin (200 U/ml) soaked gelfoam. At this phase intercostal nerves were dissected and injected with recombinant adenovirus as describe above.

In some cases, the nerves were already dissected and injected the day before and kept in DMEM to allow the virus to enter the cells overnight. The injected nerves were washed in L15 medium and three to eight nerves were gently placed as a bridge between the two stumps of the injured spinal cord, using a 80 micron glass capillary. The nerves were glued to the spinal cord using 10 µl of a two component fibrin-glue (Tissuecoll, Immuno B.V.). The lesion was covered with a non-absorbable, polyester urethane patch for dura mater substitution (Neuro-patch, B. Braun). The wound was closed in layers.

Animals received post operatively fynadine (2.5 mg\kg) and 5 ml saline to compensate for fluid loss during surgery. Bladders were manually emptied for the first 7-10 days until normal bladder function returns.

### 7. Implantation of transduced costal nerve in the sciatic nerve

Rats were anaesthetized with isofluorane and at the level of the hip, the right sciatic nerve was exposed. Referential points were created by making two small ligatures with Ethilon 10\0 silk sutures in the epineurium of the peroneal nerve approximately 4 mm distant from each other. The nerve was axotomised using sharp operating scissors between the two reference points. Six injected intercostal nerves were gently placed between the two stumps of the sciatic nerve using a 80 micron glass capillary. The nerve ends of the transplants were glued to the proximal and distal parts of the sciatic nerve, using fibrin glue. The sciatic nerve was then placed in its original position and the skin was closed using ethicon silk sutures.

### 8. Implantation into motor neuron pool after ventral root avulsion

Rats were anaesthetized with isofluorane and a laminectomy was performed at the level of Th11- L2. With micro forceps, the ventral root was gently removed from its original position in the spinal cord. An incision was horizontally made in the dura, approximately 1 mm above the avulsed root (Carlstedt et al., Lancet 346: 1323-1325 (1995)), and a 6 mm piece of transduced intercostal nerve was gently inserted in the spinal cord using a glass capillary. The distal end of the intercostal nerve was connected with the avulsed root and the stumps were fixed with 10 µl fibrin-glue. The wound was closed in layers and the animal was allowed to return to the cage.

### EXAMPLE 4

### Adeno-associated viral vector (rAAV) production and purification

rAAV is produced following CaPO₄-transfection of 5 µg of vector plasmid (based on pTR-UF) and 25 µg of helper plasmid (pAd8) on 293T cells. After 6 hr the transfection medium is removed and the cells are infected with helper virus (Ad5) in DMEM containing 10% FCS and antibiotics, at a moi of 3. At 48 hr after the infection cells are collected in 2 ml DMEM medium per 10 cm culture dish. The cells are freezed and thawed four times in a dry ice/ethanol bath and a 37°C waterbath. Subsequently, medium and cells are treated with 10 µg/ml DNase I for 30 min., followed by treatment with 1% sodium deoxycholate for 30 min. at 37°C. The medium should become clear and is mixed with 1/3 volume of saturated ammonium sulphate at 4C and incubated on ice for 10 min.

After the incubation on ice the formed precipitate is centrifugated in an SW28 rotor at 10,000 rpm for 10 min. After centrifugation the supernatant is mixed with 2/3 volume of ammonium sulphate, incubated on ice for 20 min. and centrifugated at 12,000 rpm in a SW28 rotor for 20 min. The supernatant is discarded and the pellet is dissolved in 2.5 ml of PBS. The virus solution in PBS is loaded into 5ml quick seal UC tubes (Beckman), and underlayered with an equal volume of 60% Iodixanol (Optiprep™) with a sterile Pasteurs pipet. After sealing the tube, the tube is rotated for 12 min under an angle of 80 degrees to pre-form a gradient. After pre-forming the gradient the tube is centrifugated for 4 hr in a NVT90 rotor at 71,000 rpm at 16°C.

Fractions of the gradient are collected from the tube and titered on 293 cells. The positive fraction(s) are pooled, diluted at least 6 times with PBS to dilute the Iodixanol, and concentrated on a Centricon 30 column. The solution thus obtained can be used directly for injection.

## Claims

1. Use of a viral vector other than wild type adenoviral vectors containing the gene for a nerve growth influencing molecule for the preparation of a therapeutical composition for the treatment of injuries in the peripheral or central nervous system.

2. Use as claimed in claim 1, wherein the nerve growth influencing molecule is a nerve growth stimulating molecule selected from the group consisting of neurotrophic factors, cell adhesion molecules, growth associated proteins and their receptors.

3. Use as claimed in claim 2, wherein the neurotrophic factor is selected from the group consisting of brain-derived neurotrophic factor (BDNF), neurotrophic factor 3 (NT-3), nerve growth factor (NGF), glial-derived neurotrophic factor (GDNF), acidic and basic fibroblast growth factor (FGF).

4. Use as claimed in claim 2, wherein the cell adhesion molecule is selected from the group consisting of L1, neural-cell adhesion molecule, Axonin-1/TAG-1, cadherins.

5. Use as claimed in claim 2, wherein the growth associated protein is B-50/GAP-43.

6. Use as claimed in claim 2, wherein the receptor is a member from the tyrosine-kinase receptor family, such as trkA, trkB, trkC.

7. Use as claimed in any one of the claims 1 to 6, wherein the injured neurons are the result of dorsal or ventral root avulsions.

8. Use as claimed in any one of the claims 1 to 6, wherein the injured neurons are the result of contusion of the spinal cord.

9. Use as claimed in any one of the claims 1 to 6, wherein the injured neurons display atrophy in the brain nuclei.

10. Use as claimed in any one of the claims 1 to 6, wherein the injured neurons are crushed or cut central or peripheral nerve cells.

11. Use as claimed in any one of the claims 1-10, wherein the viral vector is selected from adeno-associated viral (AAV) vectors and adenoviral vectors.

12. Use as claimed in claim 11, wherein the adeno-associated viral vectors are preferably human adeno-associated vector-2 (AAV-2).

13. Use as claimed in claim 13, wherein the adenoviral vectors are selected from the group consisting of first, second, third or further generation adenoviral vectors.

14. Method for concentrating adeno-associated viral vectors, comprising the step of banding the vectors in Iodixanol.
